# EUROPEAN PATENT APPLICATION

(11) **EP 1 681 279 A1**
(43) Date of publication of application: **19.07.2006**
(21) Application number: 04799561.8
(22) Date of filing: 02.11.2004
(51) Int. Cl.: C07C 2/22, C07C 2/30, C07C 11/107, B01J 31/36, C07B 61/00

(54) **PROCESS FOR PRODUCING 1-HEXENE**

(30) Priority: 05.11.2003 JP 2003375297; 05.11.2003 JP 2003375296; 10.03.2004 JP 2004067147; 10.03.2004 JP 2004067146
(71) Applicant: Sumitomo Chemical Company, Limited, Tokyo 104-8260 (JP)
(72) Inventor: IWAKURA, Kazunori, Toyonaka-shi Osaka 5600026 (JP); YANAGAWA, Masao, Akashi-shi Hyogo 6730003 (JP); ODA, Seiji, Ibaraki-shi Osaka 5670841 (JP)
(74) Representative: Vossius & Partner
(86) International application number: PCT/JP2004/016623
(87) International publication number: WO 2005/044761

(57) **Abstract**

There is disclosed a process for producing 1-hexene, which comprises trimerizing ethylene in the presence of a catalyst comprising a tantalum compound and an alkylating agent containing a metal under a condition wherein the water content in the reaction system is 4 moles or less per mole of the tantalum atom, and/or under a condition wherein the amount of molecular oxygen in the reaction system is 2 moles or less per mole of the tantalum atom.

## Description

### Technical Field

The present invention relates to a process for producing 1-hexene by trimerizing ethylene.

### Background Art

There is disclosed in USP 6, 344, 594 and J. Am. Chem. Soc. 2001, 123, 7423, as a process of using a tantalum compound for trimerizing ethylene, a process of using a catalyst system comprising a tantalum compound and an alkylating agent.

The disclosure of the two references was silent on the amounts of water and molecular oxygen that affect the reaction, and there has been a problem in that the reaction does not proceed at all when insufficiently dehydrated or deoxygenated catalyst, ethylene gas and solvents are used.

### Disclosure of the Invention

According to the present invention, 1-hexene can be stably produced with excellent selectivity. Thus, the present invention provides a process for producing 1-hexene, which comprises trimerizing ethylene in the presence of a catalyst comprising a tantalum compound and an alkylating agent containing a metal under a condition wherein the water content in the reaction system is 4 moles or less per mole of the tantalum atom, and/or under a condition wherein the amount of molecular oxygen in the reaction system is 2 moles or less per mole of the tantalum atom.

### Best Mode for Carrying Out the Invention

The tantalum compound used in the invention is preferably a tantalumhalide, and specific examples thereof include tantalum pentafluoride, tantalum pentachloride, tantalum pentabromide, and tantalum pentaiodide. Preferred is tantalum pentachloride or tantalum pentabromide.

The alkylating agent containing a metal which is used together with the tantalum compound is a hydrocarbyl metal, a hydrocarbyl metal halide, an alkylaluminoxane, or the like. Preferred examples thereof include lower alkyl metal compounds, aryl metal compounds, and lower alkylaryl metal compounds which contain, as their metal species, tin, zinc, aluminum, lithium or magnesium. More preferred examples thereof include tetramethyltin, tetraethyltin, dimethylzinc, methyllithium, trimethylaluminum, n-butyllithium, allyltriphenyltin, triethylaluminum, dimethylaluminum chloride, tetraphenyltin, methylaluminoxane, and methylmagnesium bromide.

These alkylating agents may be used alone or in the form of a mixture of two or more thereof.

The tantalum compound and the alkylating agent are mixed in an appropriate ratio to give good 1-hexene selectivity and catalyst activity. The molar ratio of the tantalum compound/the alkylating agent is preferably from 0 . 1 to 10, more preferably from 0.25 to 2, even more preferably from 0.5 to 2.

The mixing of the tantalum compound and the alkylating agent and the ethylene-trimerization reaction are usually conducted in the presence of a solvent. Examples of the solvent include aliphatic hydrocarbons such as butane, pentane, hexane, heptane, octane, cyclopentane, cyclohexane, or methylcyclopentane; aromatic hydrocarbons such as benzene, toluene, xylene, cumene, or ethylbenzene; halogenated aromatic hydrocarbons such as monochlorobenzene, or dichlorobenzene; halogenated hydrocarbons such as dichloromethane, chloroform, or 1, 1-dichloroethane; and olefins such as 1-butene, 1-hexene or 1-octene. In view of the activity of the catalyst and the selectivity to 1-hexene, preferred are aromatic hydrocarbons and halogenated aromatic hydrocarbons. More preferred is benzene, toluene, xylene or monochlorobenzene. These solvents may be used alone or in the form of a mixture of two or more thereof.

When the mixing of the tantalum compound and the alkylating agent and the ethylene-trimerization reaction are carried out, the concentration of the tantalum compound in the solvent is not particularly limited, and is usually from 0.0001 µmol to 100 mmol, pref-erably from 0.001 µmol to 10 mmol per liter of the solvent.

The catalyst used in the ethylene-trimerization reaction in the invention is a catalyst comprising the tantalum compound and the alkylating agent containing a metal, and can be prepared by mixing the tantalum compound with the alkylating agent. The method for the preparation is not particularly limited, and is, for example; a method of mixing the tantalum compound with the alkylating agent in the absence of any solvent or in the above-mentioned solvent. The mixing of the tantalum compound with the alkylating agent is beforehand conducted, and subsequently the mixture is brought into contact with ethylene, ' whereby the trimerization can be carried out. The mixing of the tantalum compound with the alkylating agent is conducted in the presence of ethylene, whereby the mixing of the tantalum compound with the alkylating agent and the trimerization can be simultaneously started. The mixing order of these starting materials is not particularly limited. Preferably, the alkylating agent is added to the tantalum compound in view of the activity of the catalyst and the selectivity to 1-hexene.

The temperature for the trimerization reaction of the present reaction is usually from -50 to 250°C, preferably 200°C or lower, more preferably from 0 to 150°C, even more preferably from 10 to 100°C. The reaction pressure is usually an absolute pressure ranging from 0 to 300 MPa, preferably from 0.1 to 30 MPa.

The invention is carried out under a condition where the water content in the reaction system is 4 moles or less , preferably 3 moles or less, more preferably 2 moles or less per mole of the tantalum atom, even more preferably under a substantially anhydrous condition (the water concentration in this case is a concentration lower than the detectable limitation of water, and is a concentration of about 5 ppm or less), and/or under a condition where the amount of molecular oxygen in the reaction system is 2 moles or less, preferably 1.5 moles or less, more preferably 1 mole or less per mol of the tantalum atom, even more pref erably under a substantially oxygen-free condition (the oxygen concentration in this case is a concentration lower than the detectable limitation of oxygen, and is a concentration of about 1 ppm or less).

The water content in the reaction system is the total amount of water present in the gas phase and the liquid phase in the reaction system. The water content in the gas phase can be measured with a dew point hygrometer, and the water content in the liquid phase can be measured with a Karl Fisher moisture meter. The water content in the reaction system may be usually represented by the water content in the liquid phase as long as there are neither effects based on difference between methods for the reaction nor other especial circumstances.

The amount of molecular oxygen in the reaction system is the total amount of molecular oxygen in the gas phase and the liquid phase in the reaction system. The amount of molecular oxygen in the gas phase can be measured by an oxygen densitometer. The amount of molecular oxygen in the liquid phase can be measured by gas chromatography. The amount of molecular oxygen in the reaction system may be usually represented by the amount of molecular oxygen in the liquid phase as long as there are neither effects based on difference between methods for the reaction nor other especial circumstances.

Examples of the method for controlling the water content in the reaction system in the ethylene-trimerization reaction of the present invention include a method of treating the catalyst, ethylene gas, the solvent and so on with a dehydrating agent before the trimerization reaction, and a method of distilling the solvent. Any dehydrating agent that does not produce an adverse effect on the reaction can be used, and examples thereof include a substance of a single element of Groups 1 and 2 of the periodic table of elements such as lithium, sodium, potassium, magnesium, or calcium; hydrides of an element of Group 1, 2 or 13 of the periodic table such as lithium hydride, sodium hydride, potassium hydride, calcium hydride,or aluminum hydride;organic metal compounds of an element of Group 1, 2, 12 or 13 of the periodic table such as methyllithium, dimethylzinc, trimethylaluminum, n-butyllithium, triethylaluminum, dimethylaluminum chloride, methylaluminoxane, or methylmagnesium bromide; oxides of an element of Group 1, 2, 13, 14 or 15 of the periodic table such as lithium oxide, sodium oxide, potassium oxide, magnesium oxide, calcium oxide, zinc oxide, aluminum oxide, silicon oxide, or phosphorus pentaoxide; halides of an element of Group 2, 4, 12,13, 14, 15 or 16 of the periodic table such as magnesium chloride, calcium chloride, titanium chloride, zinc chloride, aluminum chloride, tin chloride, phosphorus trichloride, or thionyl chloride; and zeolites such as molecular sieves . These drying agents may be used alone or in the form of a mixture of two or more thereof.

Examples of the method for controlling the amount of molecular oxygen in the reaction system at the time of the ethylene-trimerization reaction in the range of the present invention include a method of treating the catalyst, ethylene gas, the solvent and so on with a deoxidizer before the trimerization reaction, and a method of distilling the solvent. Any deoxidizer which does not produce a bad effect on the reaction may be used, and examples thereof include a substance of a single element of Group 1, 2, 8, 10, 11, 12 or 13 of the periodic table such as lithium, sodium, potassium, magnesium, calcium, iron, nickel, palladium, platinum, copper, zinc, and aluminum; ceria; and zirconia. These deoxidizers may be used alone or in the form of a mixture of two or more thereof. The amount of molecular oxygen can be also reduced by freezing deaeration or some other operation.

The present reaction can be carried out in a batch-wise, semi-continuous manner, or a continuous manner. After completion of the reaction, an inactivating agent such as water, an alcohol, hydrochloric acid, or an aqueous sodium hydroxide solution, is added to the reaction solution, thereby terminating the reaction. After the termination of the reaction, desired 1-hexene can be separated by a known operation such as distillation or extraction. Desired 1-hexene can be separated by distillation without terminating the reaction.

### Examples

The present invention will be described in more detail by way of the following examples. However, the invention is not limited to these examples.

### Examples 1 to 13

Under nitrogen atmosphere, water-containing toluene (water content: 163 ppm) in each amount shown in Table 1 was charged into an autoclave, and thereto was added dehydrated toluene (water content: 8 ppm, and oxygen content: < 1 ppm) to prepare a solution of 4.2 mL. A gas-tight syringe was used to add oxygen gas thereto in each amount shown in Table 1 at atmospheric pressure. The temperature was stabilized at 40°C. Thereafter, the pressure of ethylene was increased up to 0.6 MPa, and stabilized. Into the solution were charged 0.5 mL of a solution of 25.1 mg of tantalum pentachloride dissolved in 7 mL of dehydrated toluene (the amount of tantalum pentachloride: 5 µmol) and 286 µL (5 µmol) of a solution obtained by diluting dimethylzinc (manufactured by Aldrich, 2 mol/L-solution in toluene) into 0.175 M with dehydrated toluene, and were reacted for each period of time shown in Table 1 . The reaction container was cooled to room temperature. Next, the pressure therein was returned to an atmospheric pressure. The reaction solution was analyzed by gas chromatography. A solid contained in the reaction solution was filtrated off with filter paper, and the solid was air-dried and then dried at a reduced pressure. The weight thereof was then measured. The results are shown in Table 1.

In the above-mentioned examples, the water content in the liquid phase was measured with a Karl Fisher moisture meter (Hiranuma AQ-6, Karl Fisher electricity quantity method, generation liquid: HydranalCoulomatAG (manufactured by Hayashi Pure Chemical Ind., Ltd.), counter electrode liquid: Hydranal Coulomat CG (manufactured by Hayashi Pure Chemical Ind. , Ltd.)). The water content in the gas phase was measured with a dew point hygrometer. As a result, it was proved that the water content was 1 ppm or less.

The amount of molecular oxygen in the liquid phase was measured with a gas chromatography (Shimadzu GC-14A, detector: TCD, column: Molecular sieves 5A). The amount of molecular oxygen in the gas phase was measured with an oxygen densitometer. As a result, it was proved that the amount was 1 ppm or less.

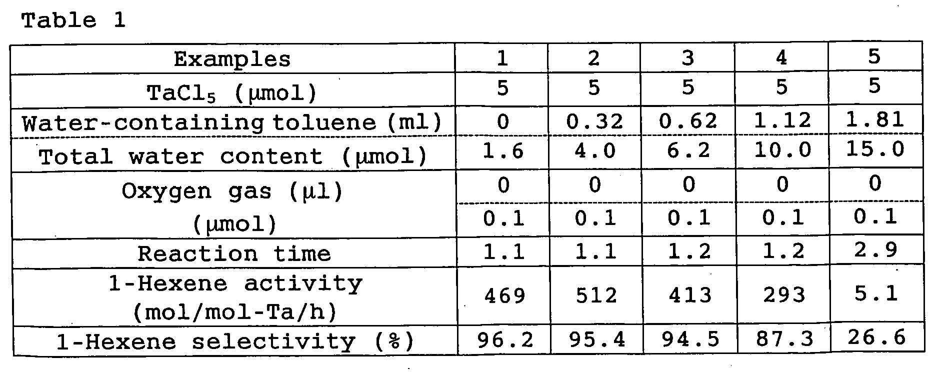

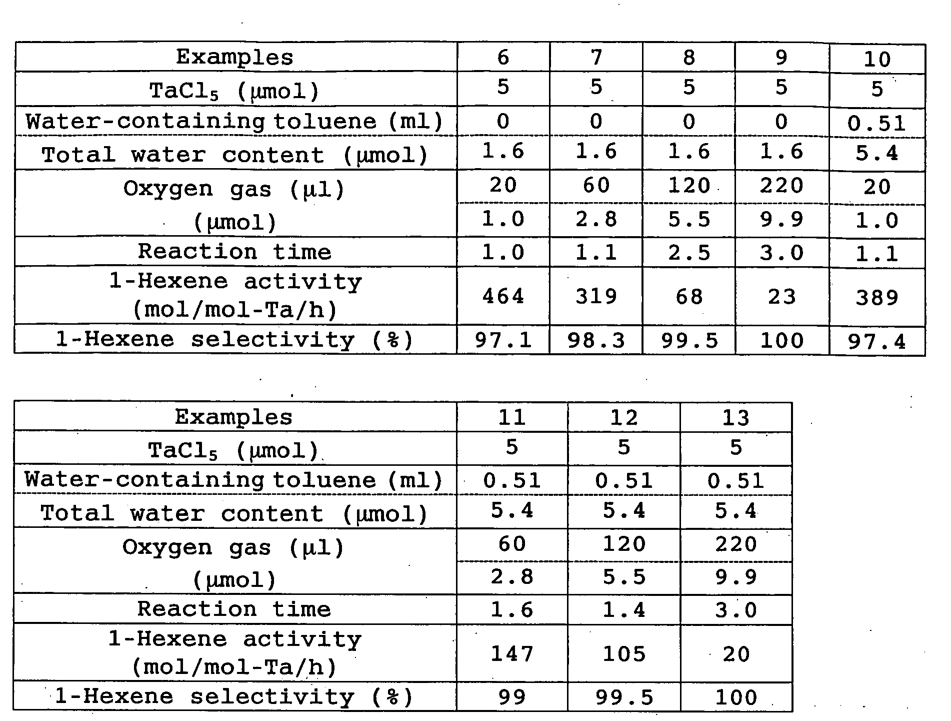

### Industrial Applicability

According to the process of the present invention, 1-hexene can stably be produced with an excellent selectivity.

## Claims

1. A process for producing 1-hexene, which comprises trimerizing ethylene in the presence of a catalyst comprising a tantalum compound and an alkylating agent containing a metal under a condition wherein the water content in the reaction system is 4 moles or less per mole of the tantalum atom, and/or under a condition wherein the amount of molecular oxygen in the reaction system is 2 moles or less per mole of the tantalum atom.

2. The process for producing 1-hexene according to claim 1, wherein the water content in the reaction system is 3 moles or less per mole of the tantalum atom.

3. The process for producing 1-hexene according to claim 1, wherein the water content in the reaction system is 2 moles or less per mole of the tantalum atom.

4. The process for producing 1-hexene according to claim 1, wherein the water content in the reaction system is substantially anhydrous.

5. The process for producing 1-hexene according to any one of claims 1 to 4, wherein the amount of the molecular oxygen in the reaction system is 1. 5 moles or less per mol of the tantalum atom.

6. The process for producing 1-hexene according to any one of claims 1 to 4, wherein the amount of the molecular oxygen in the reaction system is 1 mol or less per mole of the tantalum atom.

7. The process for producing 1-hexene according to any one of claims 1 to 4, wherein the amount of the molecular oxygen in the reaction system is substantially oxygen-free.

8. The process for producing 1-hexene according to any one of claims 1 to 7, wherein the tantalum compound is a tantalum halide.

9. The process for producing 1-hexene according to any one of claims 1 to 7, wherein the tantalum compound is tantalum pentachloride or tantalum pentabromide.

10. The process for producing 1-hexene according to any one of claims 1 to 9, wherein the alkylating agent is a hydrocarbyl metal, a hydrocarbyl metal halide, or an alkylaluminoxane.

11. The process for producing 1-hexene according to any one of claims 1 to 9, wherein the alkylating agent is tetramethyltin, tetraethyltin, dimethylzinc, methyllithium, trimethylaluminum, n-butyllithium, allyltriphenyltin, triethylaluminum, dimethylaluminum chloride, tetraphenyltin, methylaluminoxane, or methylmagnesium bromide.
